# EUROPÄISCHE PATENTSCHRIFT

(11) **EP 1 253 396 B1**
(45) Veröffentlichungstag und Bekanntmachung des Hinweises auf die Patenterteilung: **25.05.2005**
(21) Anmeldenummer: 02013632.1
(22) Anmeldetag: 06.10.1994
(51) Int. Cl.: F41A 3/54, F41A 3/26, F41A 9/32, F41A 9/33, F41A 3/78

(54) **Selbstlade-Granatwerfer**
Automatic grenade launcher
Lanceur-grenades automatique

(30) Priorität: 08.10.1993 DE 4334412
(43) Veröffentlichungstag der Anmeldung: 30.10.2002
(62) Teilanmeldung aus: 98113916.5
(73) Patentinhaber: Heckler & Koch GmbH, 78727 Oberndorf/Neckar (DE)
(72) Erfinder: Weichert, Berthold, 78658 Zimmern (DE); Gielke, Gerhard, 78727 Oberndorf (DE); Wössner, Ernst, 72172 Sulz (DE); Gablowski, Jürgen, 78727 Oberndorf (DE)
(74) Vertreter: von Samson-Himmelstjerna, Friedrich R.

(56) Entgegenhaltungen:
- DE-A- 2 016 281
- DE-C- 700 629
- GB-A- 500 922
- US-A- 2 979 992
- US-A- 3 363 351
- US-A- 4 942 802

## Beschreibung

Die Erfindung betrifft einen Selbstlade-Granatwerfer mit den Merkmalen des Oberbegriffs des Anspruchs 1.

Ein solcher Granatwerfer ist in der Zeitschrift "International Defense Review", Band 22, Nr, 12/1989 beschrieben. Er weist eine Patronengurt-Zuführeinrichtung auf, die die jeweils vorderste Patrone unmittelbar hinter das Patronenlager fördert. Ein Masseverschluß, der in der Wirkungsweise dem einer Maschinenpistole ähnelt, wird durch eine Schließfederanordnung gegen diese vorderste Patrone gefördert, schiebt sie in das Patronenlager und zündet sie.

Im folgenden werden Begriffe wie "vorne", "hinten", "seitlich" usw. ohne nähere Definition verwendet. Sie beziehen sich stets auf die in horizontaler Feuerstellung befindliche Waffe, wobei "vorne" die Mündung, also das in Schußrichtung vorderste Ende der Waffe, bedeutet.

Um den Granatwerfer feuerbereit zu machen, genügt es, den Masseverschluß gegen die Wirkung der Schließfederanordnung in seine hinterste Lage zu bewegen, in welcher er von einer Abzugseinrichtung festgehalten wird, und den Patronengurt in die Zuführeinrichtung einzulegen.

In diesem einfachen Bewegungsablauf liegt der besondere Vorteil des gattungsbildenden Granatwerfers gegenüber dem bisher am weitesten verbreiteten Selbstlade-Granatwerfer, dem US Mark 19: bei diesem muß nämlich nach dem Einlegen des Gurtes der Verschluß zunächst einmal leer abgeschlagen und dann wieder gespannt werden, da die vorderste Patrone des Gurtes beim ersten Abschlagen des Verschlusses nicht unmittelbar in das Patronenlager, sondern zunächst in eine Übergabeposition gefördert wird, aus welcher sie durch das zweite Abschlagen des Verschlusses in das Patronenlager gefördert und dort gezündet wird.

Es ist eine allgemeine Regel, daß man Schußwaffen aller Art nicht leer, also ohne Patrone oder Pufferpatrone im Patronenlager, abschlagen lassen soll.

Andererseits ist es bei der Ausbildung an den Waffen erforderlich, das Schießen, Ladegriffe usw. häufig zu üben. Um die Beschädigung des erfindungsgemäßen Granatwerfers zu vermeiden, die er durch das leere Abschlagen des Masseverschlusses erleiden könnte, ist eine Pufferfedereinrichtung vorgesehen, die den Masseverschluß in der letzten Phase des Vorlaufes abfängt und abbremst, um einen Dauerschaden an der Waffe zu verhindern. Eine solche Pufferanordnung ist an sich durch die GB-A-500 922 bekannt.

Aufgabe der Erfindung ist es, eine wirksame Pufferaktion bei geringem Fertigungsaufwand zu liefern.

Gelöst wird diese Aufgabe durch den Gegenstand des einzigen Patentanspruches.

Im Gegensatz zur GB-A-500 922 ist die gemäß der Erfindung vorgesehene Pufferfedereinrichtung so angeordnet, daß sie beim normalen Schießvorgang nicht in Wirkung tritt, da dann der Masseverschluß allenfalls bis zu dem ein Stück weit aus dem Patronenlager herausstehenden Boden der Patrone vorlaufen kann, nicht aber bis zur Pufferfedereinrichtung.

Außerdem sind zur Lösung dieser Aufgabe zwei sich parallel zur Waffenlängsachse erstreckende Federführungsstäbe für die Schließfedern vorgesehen, die den Masseverschluß jeweils in einer Längsbohrung durchsetzen. Am vorderen Ende dieser Federführungsstäbe ist jeweils eine Pufferfeder angeordnet, die gegen das vordere Ende des Gehäuses abgestützt ist und bevorzugt teilweise in einer Bohrung sitzt, damit ein hinlänglicher Raum für deren Aufnahme geschaffen ist.

Die Anordnung zweier Pufferfedern liefert wie auch die Anordnung zweier Schließfedern eine Redundanz von Teilen, die im Falle des Brechens einer Feder einen weiteren Betrieb der Waffe, wenn auch vielleicht unter Störungen, ermöglicht.

Die Pufferfedern können gegebenenfalls nur für Ausbildungszwecke eingebaut sein, vor dem Einsatz der Waffe aber ausgebaut werden.

Der Gegenstand der Erfindung wird anhand der beigefügten, schematischen Zeichnung beispielsweise noch näher erläutert, in der eine einzige bevorzugte Ausführungsform des erfindungsgemäßen Granatwerfers gezeigt ist. In dieser Ausführungsform sind alle oben aufgeführten Merkmale der Ansprüche vereinigt.

Es wird jedoch ausdrücklich darauf hingewiesen, daß die zusammengehörigen Gruppen dieser Merkmale auch unabhängig von anderen Merkmalsgruppen bei einer Waffe realisiert werden können.

In der Zeichnung zeigt:
- Fig. 1: den Längs-Aufriß eines Ausführungsbeispiels eines erfindungsgemäßen Granatwerfers, mit abgenommenem Gehäusedeckel und Zubringerdeckel,
- Fig. 2: eine Draufsicht auf den in Fig. 1 gezeigten Granatwerfer,
- Fig. 3: einen schematischen Schnitt durch den Granatwerfer längs Linie III - III in Fig. 1,
- Fig. 4: einen Schnitt ähnlich dem der Fig. 3, wobei die Zubringerstellung nach Einlegen des Patronengurtes dargestellt ist, wobei sich der Masseverschluß in seiner Auslöselage (hintersten Lage) befindet,
- Fig. 5: einen Schnitt wie in Fig. 4, nachdem der Masseverschluß seine Vorwärtsbewegung begonnen hat,
- Fig. 6: einen Schnitt wie in Fig. 4, wobei sich die vorderste Patrone in Zuführposition befindet,

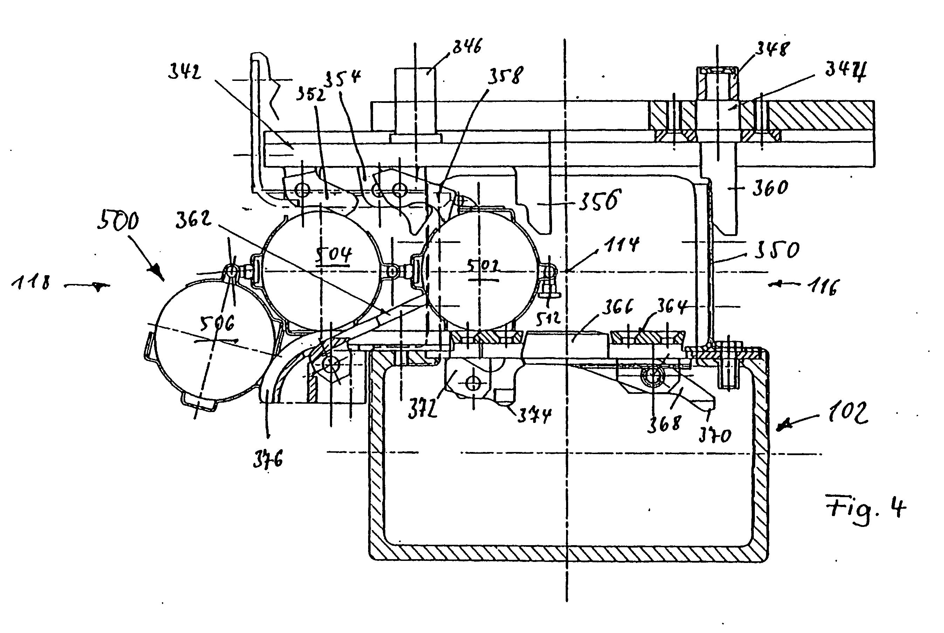

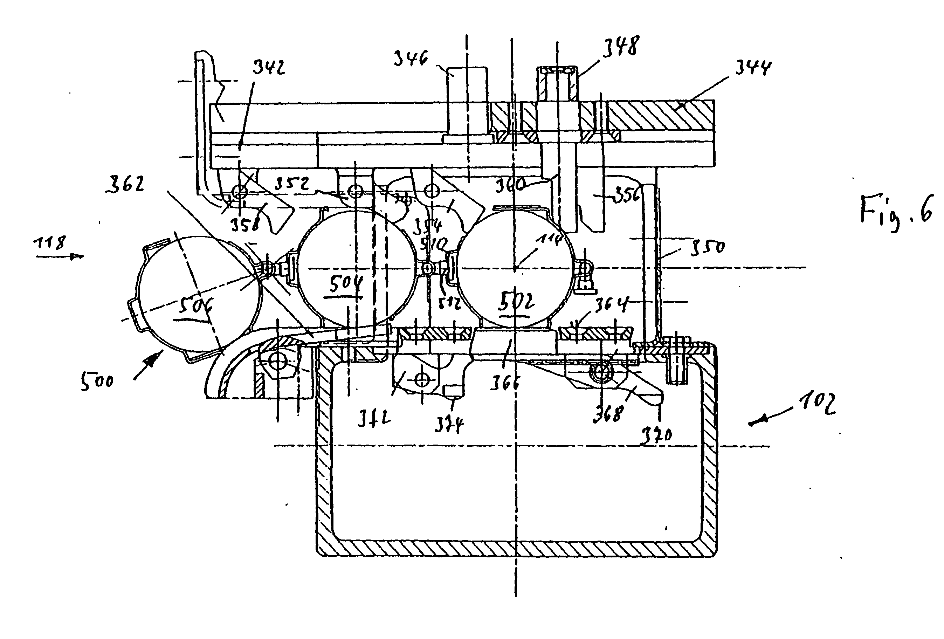

- Fig. 6: einen Schnitt wie in Fig. 4, bei Zündung der Patrone,

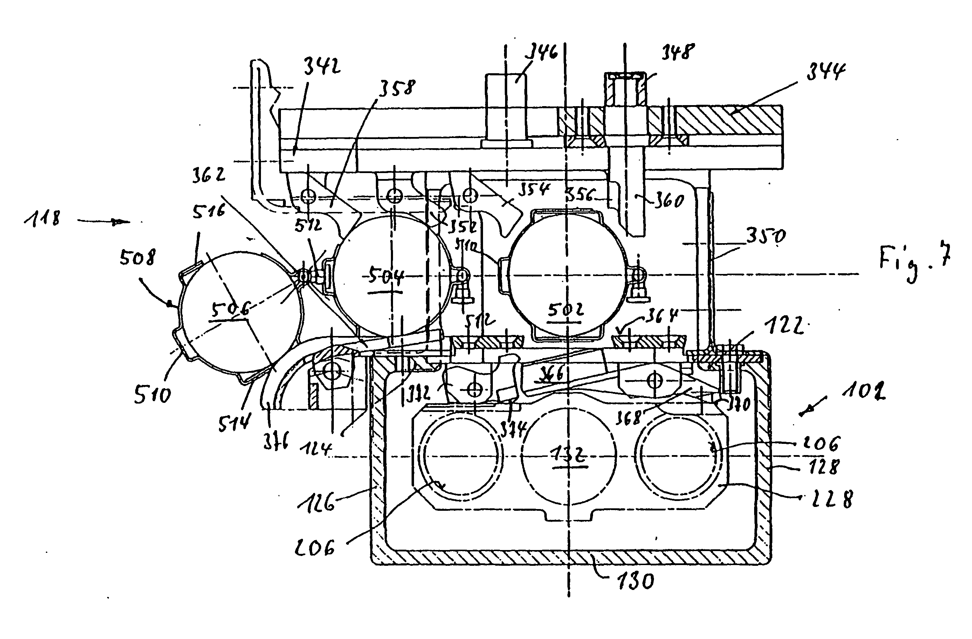

- Fig. 7: einen Schnitt wie in Fig. 4, bei Beginn des Rücklaufes des Masseverschlusses,

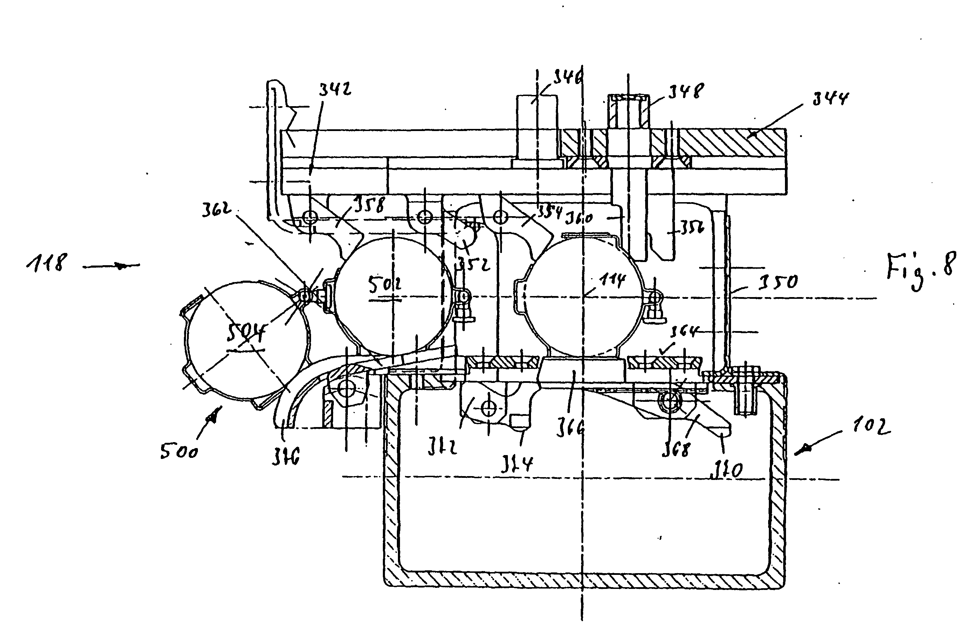

- Fig. 8: einen Schnitt wie in Fig. 4, beim Ausziehen der abgefeuerten Patronenhülse,

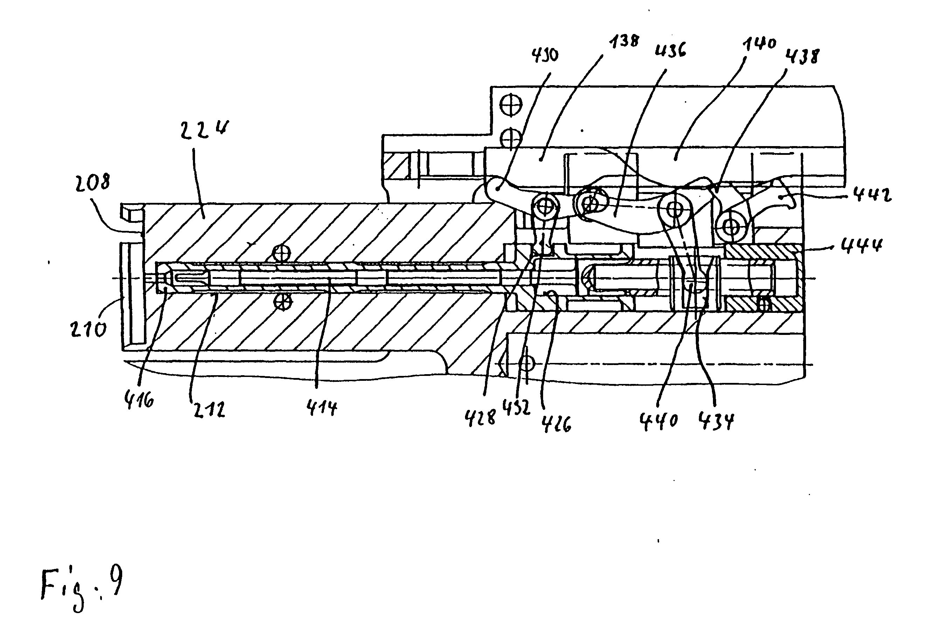

- Fig. 9: einen Teilschnitt durch den Verschlußkopf des Masseverschlusses, und

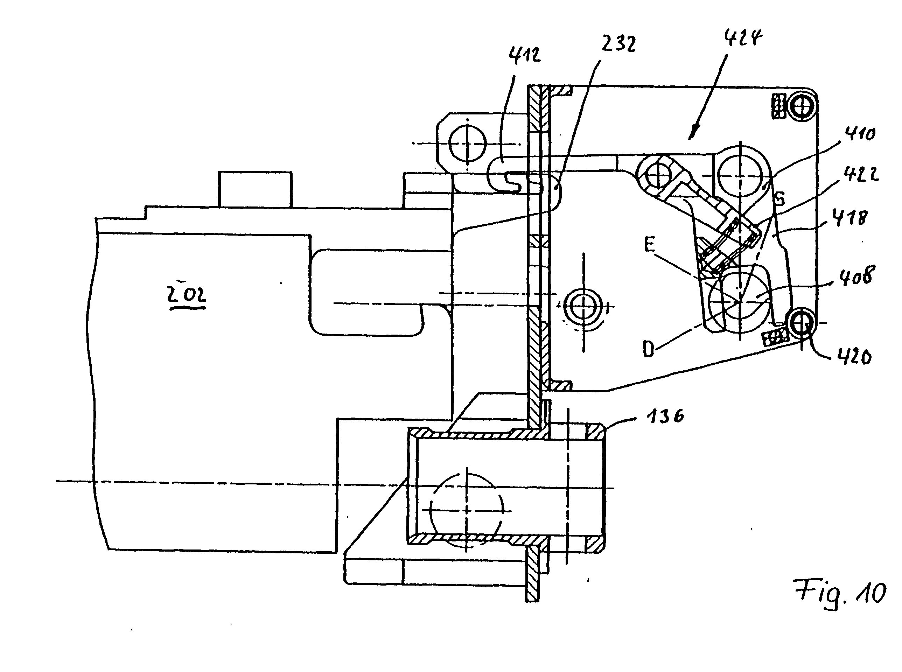

- Fig. 10: eine schematische, teilweise geschnittene Darstellung der Verschlußfangeinrichtung.

In den Figuren bedeuten gleiche Bezugszeichen durchgehend gleiche Bauteile oder Elemente.

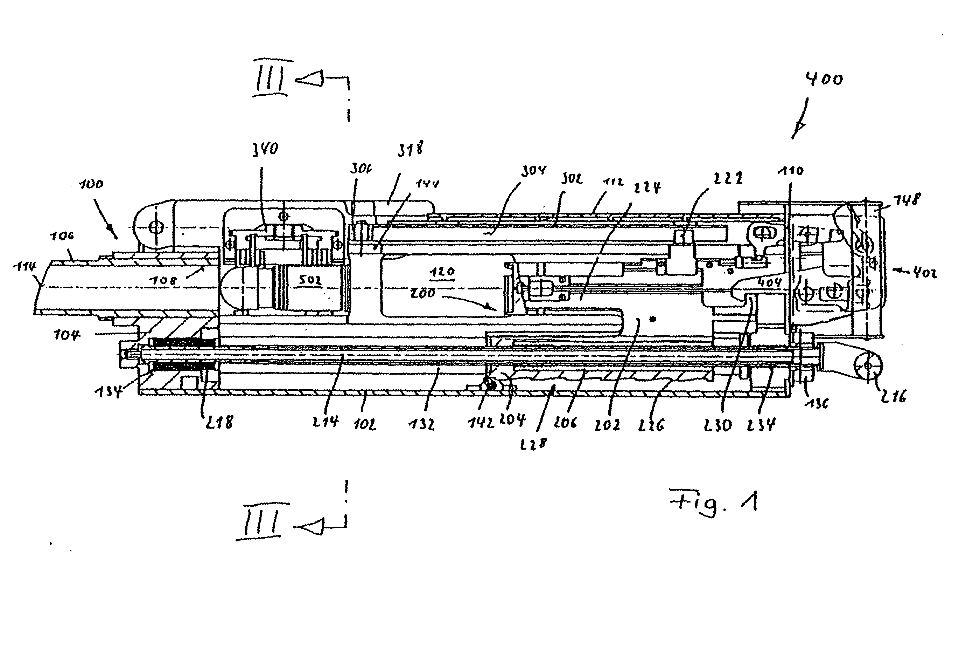

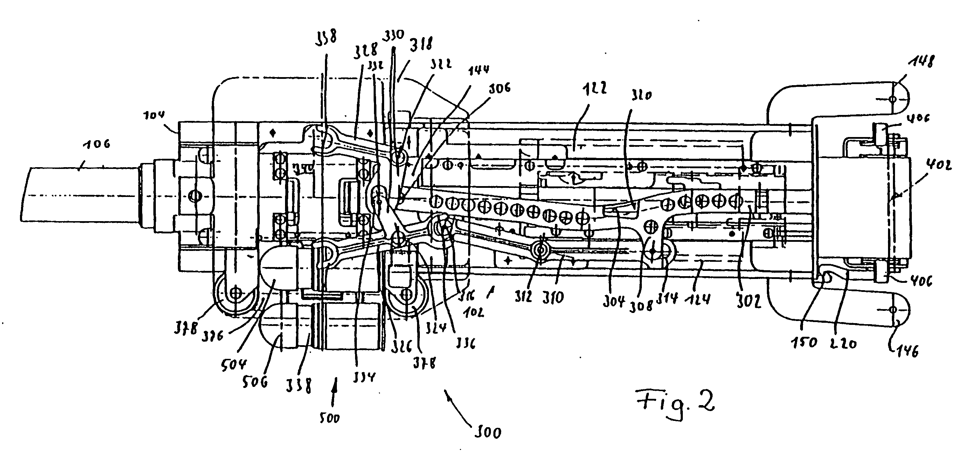

Der in den Übersichtsdarstellungen der Fig. 1 und 2 gezeigte Granatwerfer besteht im wesentlichen aus einer Gehäusegruppe 100, einer Verschlußgruppe mit Federung und Handhabungseinrichtung 200, einer Zubringergruppe mit Steuerung 300 und einer Abzugseinrichtungsgruppe 400. Der in den Granatwerfer eingeführte Patronengurt ist mit 500 bezeichnet, ist an sich bekannt und bildet als solcher keinen Bestandteil der Waffe.

### Der Patronengurt 500:

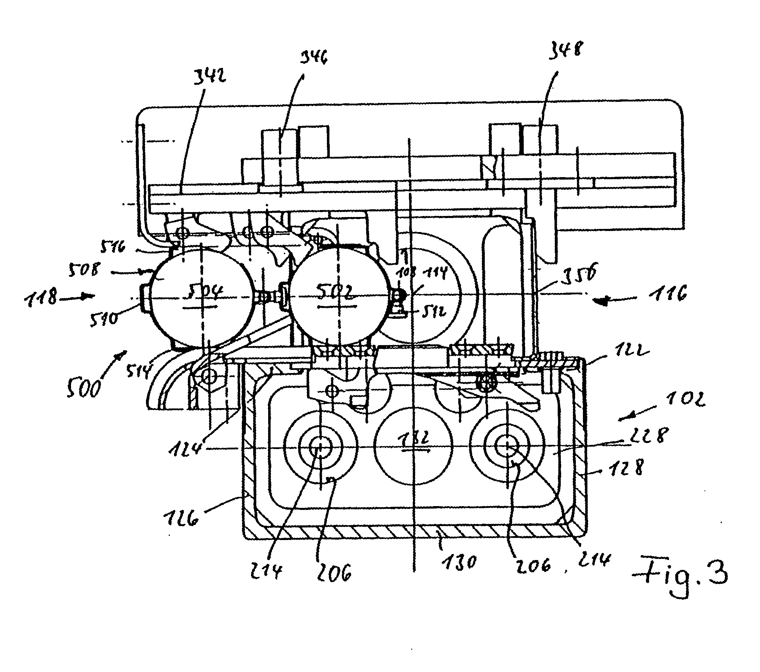

Zum vollen Verständnis der Waffe soll aber zunächst der bekannte Patronengurt in Erinnerung gerufen werden, wobei auf die Figuren 1 und 3 Bezug genommen wird. In den übrigen Figuren der Zeichnung sind die den Patronengurt betreffenden Bezugszeichen weggelassen, um nicht zu verwirren.

Der Patronengurt enthält eine vorderste Patrone 502, eine erste nachfolgende Patrone 504 und weitere Patronen 506 (nur eine in Fig. 2 gezeigt).

Die Patronen 502, 504 und 506 weisen jeweils ein Geschoß und eine Patronenhülse auf, die am hinteren Ende einen flanschartig überstehenden Rand aufweist sowie den Zündsatz und die Treibladung aufnimmt.

Jede Patronenhülse trägt ein sie wie eine Rohrschelle umschließendes, aus einen Blechband gebildetes Gurtglied 508. In Fig. 2 sind die Gurtglieder 508 weggelassen.

Das Gurtglied weist an der Ober- und Unterseite der Patrone 502, 504 jeweils einen breiten, abgeflachten Vorsprung 516 bzw. 514 auf, auf der einen (in Fig. 3 linken) Seite einen schmalen, abgeflachten Vorsprung 510, der ein Langloch mit erweitertem Ende aufweist, und auf der anderen Seite einen Vorsprung mit einem gelenkig an diesem angebrachten Anlenkzapfen 512, der ein verdicktes freies Ende aufweist.

Der Anlenkzapfen 512 sitzt bei dem zusammengesetzten Gurt im Langloch des Vorsprungs 510 des benachbarten Gurtgliedes 508 und hintergreift dieses mit seinem verdickten Ende.

Werden benachbarte Patronen gegeneinander verschoben, dann gelangt das verdickte Ende des Anlenkzapfens 512 vor das erweiterte Ende des ihn aufnehmenden Langloches, so daß die beiden benachbarten Patronen 502, 504 auseinanderbewegt werden können. So erfolgt das Entgurten; das Gurtglied 508 verbleibt auch an der entgurteten Patrone.

Das Gurtglied 508 sitzt bei der unabgeschossenen Patrone etwa an der vorderen Hälfte der Patronenhülse und umschließt diese stramm.

Wird die Patrone in ein Patronenlager 108 eingeführt, dann sitzt das Gurtglied auf dem hinteren Ende des Patronenlagers 108 auf und wird bis gegen den Patronenrand nach hinten geschoben. Die Patrone läßt sich somit nur soweit in das Patronenlager 108 einführen, daß der Patronenrand um einen Abstand vom hinteren Ende des Patronenlagers 108 getrennt ist, der der axialen Länge des Gurtgliedes 508 entspricht.

Die Patronenhülse ist so aufgebaut, daß sie dem Gasdruck beim Abfeuern standhält, obwohl sie nicht gänzlich in das Patronenlager 108 eingeführt ist.

Es folgt nun die Beschreibung des bevorzugten Ausführungsbeispiels des erfindungsgemäßen Granatwerfers:

### Die Gehäusegruppe 100:

Das Hauptteil der Gehäusegruppe ist von einer stranggepreßten Hohlprofilstange 102 gebildet, die im folgenden kurz "Gehäuse" genannt wird und die im wesentlichen einen Querschnitt mit zwei parallelen Seitenschenkeln aufweist, die einstückig an ihrem unteren Ende und etwa in ihrer Mitte durch jeweils einen geraden, rechtwinklig angesetzten Quersteg verbunden sind.

Das Gehäuse 102 weist somit eine linke Gehäusewand 126, eine rechte Gehäusewand 128 und einen Gehäuseboden 130 auf.

Die Längsmittellinie oder Längsmitte des Gehäuses ist mit 114 bezeichnet.

Das Gehäuse 102 wird durch Ablängen und nachfolgendes spanendes Bearbeiten einer stranggepreßten Hohlprofilstange gebildet, wobei infolge der spanenden Bearbeitung eine vordere, querverlaufende Einfräsung gebildet ist, welche zum Einführen des Patronengurtes 500 dient, mit einer rechten Einführöffnung 116 und einer linken Einführöffnung 118, ferner eine in der rechten Gehäusewand ausgearbeitete Auswurföffnung 120, durch welche abgeschossene Patronenhülsen, Exerzierpatronen oder Patronenversager aus dem Gehäuse ausgeworfen werden, und eine längsverlaufende Einfräsung im oberen Quersteg, so daß von diesem ein in Fig. 2 und 3 gezeigter, rechter Gehäusesteg 122 und linker Gehäusesteg 124 gebildet ist; an jeder der einander zugewandten Kanten der beiden Gehäusestege 122, 124 ist jeweils eine Stahlleiste mit einer Steuerkurve angebracht, und zwar die Steuerkurve 138 für die Schlagbolzenhülse 416 an der rechten Kante und die Steuerkurve 140 zum Steuern des Abschlagens des Schlagbolzens 414 an der linken Kante.

Dort, wo die von den beiden Gehäusestegen 122, 124 begrenzte Aussparung im oberen Quersteg nicht erforderlich ist, bleibt dieser stehen, etwa bei der Brücke 144.

Das Gehäuse 102 ist hartanodisiert, um eine geeignete Einfärbung (Tarnfarbe) und Oberflächengüte (Abriebbeständigkeit und Gleitverhalten) zu erhalten.

Im vorderen Ende des Gehäuses 102 ist ein Stahlblock 104 fest angebracht, der das auf die Längsmitte 114 zentrierte Rohr 106 mit dem Patronenlager 108 trägt.

Der Stahlblock 104 weist unterhalb und beiderseits des Rohres 106 jeweils eine nach hinten offene Sack-Aufnahmebohrung 134 auf, die jeweils von einem Federführungsstab 214 durchsetzt ist und eine auf diesem sitzende und abgestützte Pufferfeder 218 aufnimmt.

Die Pufferfeder reicht nach hinten bis in die vergrößerte Mündung der Aufnahmebohrung 134; diese Mündung ist so bemessen, daß sie jeweils einen Vorsprung 204 des Verschlußträgers 228 des Masseverschlusses 202 aufnehmen kann, wenn sich dieser bis ganz nach vorne bewegt (beim Abschlagen ohne Patrone).

In den Boden der Sackbohrung 134 mündet eine abgesetzte Abstütz- und Aufnahme-Durchgangsbohrung ein, in der der mit einer Führungsringwulst und einem Endzapfen ausgebildete Federführungsstab 214 im wesentlichen spielfrei aufgenommen ist. Hierbei ist das freie, vordere Ende des Endzapfens abgerundet, so daß der Federführungsstab, wenn er nach vorne in die Aufnahmebohrung 134 bewegt wird, sich selbst ausrichten kann.

In der Mitte zwischen den beiden Aufnahmebohrungen ist das Gehäuse 102 der Länge nach von einem Paß-Rundstab 132 (Fig. 1, angedeutet in Fig. 3) durchsetzt, der im Stahlblock 104 befestigt ist und der den Masseverschluß 202 bei seiner Bewegung führt.

An der Rückseite des Gehäuses 102 ist dieses durch eine Endabdeckung 110 verschlossen, in der zwei Führungen 136 für die Federführungsstäbe 214 sitzen und in der der Paß-Rundstab 132 abgestützt ist.

Die Endabdeckung trägt einen Teil der Abzugseinrichtungsgruppe 400 und ist zusammen mit dieser und der Verschlußgruppe 200 nach hinten abnehmbar.

Die Oberseite des Gehäuses 102 ist von einem abnehmbaren Gehäusedeckel 112 abgedeckt, der sich von der Endabdeckung 110 bis etwa zur Brücke 144 erstreckt.

Etwa in der Mitte der Länge des Gehäuses 102 ist an der Innenseite des Gehäusebodens 130 eine Ratschenklinke 142 um eine Querachse schwenkbar angebracht und durch eine Federung (nicht gezeigt) so belastet, daß sie danach trachtet, eine im wesentlichen aufrechte Lage einzunehmen.

Am Gehäuse 102 sind noch weitere, hier im einzelnen nicht dargestellte Teile befestigt, etwa ein Ausstoßer an der Innenseite der linken Gehäusewand 126, eine Montage für eine Visiereinrichtung an der Außenseite der linken Gehäusewand 126, jeweils eine Halterung für einen Patronenkasten außen an der linken oder rechten Gehäusewand 126, 128 im Bereich der linken und rechten Einführöffnung 118, 116, eine Halterung zum Anbringen einer Lafette außen an der linken und rechten Gehäusewand 126, 128 und/oder am Gehäuseboden 130 usw.

Außerdem ist am hinteren Ende außen an der linken und rechten Gehäusewand 126, 128 jeweils ein oberer und unterer, sich nach hinten und außen erstreckender Haltebügel angebracht; die Enden der übereinanderliegenden Haltebügel sind jeweils durch einen insgesamt vertikalen linken bzw. rechten Handgriff 146, 148 verbunden.

Das Ergreifen eines oder beider Handgriffe 146, 148 erlaubt das Richten und Feuern des Granatwerfers in üblicher Weise.

Schließlich ist an der Rückseite der linken Gehäusewand 126 unten und außen ein sich nach hinten erstreckender Fortsatz angebracht, der an seinem hinteren Ende einen einwärts weisenden Rastvorsprung 150 aufweist, aber insgesamt so angebracht ist, daß er das Entnehmen und Einsetzen der Verschlußgruppe 200 nicht behindert.

### Die Verschlußgruppe 200:

Die Verschlußgruppe 200 weist einen Masseverschluß 202 auf, der aus einem zur Längsmitte 114 koaxialen Verschlußkopf 224 und einem zu dieser parallelen Verschlußträger 228 gebildet ist, die übereinanderliegen und an ihrer Rückseite einstückig miteinander verbunden sind.

Der Verschlußkopf 224 weist an seiner Vorderseite einen Stoßboden 208 auf, der an der rechten Seite von einem üblichen, abgefederten, nach vorne überstehenden Auszieher 210 begrenzt ist.

Diesem gegenüberliegend ist auch an der linken Seite ein Auszieher (nicht gezeigt) angeordnet, um auch bei Erschütterungen der Waffe ein störungsfreies Ausziehen der Patronenhülse durch den vom Patronengurt 500 eingenommenen Bereich hindurch bis vor die Auswurföffnung 120 sicherzustellen; dieser linke Auszieher wird beim Verschlußrücklauf durch einen gehäusefesten Anschlag geöffnet und gibt den Rand der ausgezogenen Patronenhülse frei, kurz bevor dieser gegen den ebenfalls gehäusefesten Ausstoßer aufläuft.

Der Verschlußkopf 224 weist koaxial zur Längsmitte 114 eine Axialbohrung 212 auf (sh. Fig. 7), die als nach hinten offene Sackbohrung ausgebildet ist, deren Boden in üblicher Weise von einem Durchtrittskanal für die Spitze des Schlagbolzens 414 durchsetzt ist.

Diese Axialbohrung 212 nimmt die schon oben angesprochene Schlagbolzenhülse 416, den Schlagbolzen 414 und dessen Schlagfeder (nicht gezeigt) auf.

Der Verschlußträger 228 weist drei Bohrungen auf: eine (nicht gezeigte) Paßbohrung, die dazu eingerichtet ist, im wesentlichen spielfrei auf dem Paß-Rundstab 132 zu gleiten, und zwei nach hinten offene Schließfeder-Aufnahme-Sackbohrungen 206, die zu jeweils einer der Aufnahmebohrungen 134 koaxial sind.

Der Boden dieser Schließfeder-Aufnahme-Sackbohrungen 206 ist jeweils von einer kleineren Bohrung durchsetzt, durch die sich jeweils ein Federführungsstab 214 hindurch erstreckt.

Auf den hinteren Abschnitt eines jeden Federführungsstabes 214 ist jeweils eine Schließfeder 234 aufgeschoben, die als wendelförmige Druckfeder ausgebildet ist.

Jede dieser Schließfedern stützt sich vorne gegen den Boden der zugehörigen Schließfeder-Aufnahmebohrung 206 und hinten gegen die Federstabführung 136 ab.

An der Vorderseite des Verschlußträgers 228 sind die schon oben erwähnten Vorsprünge 204 ausgebildet.

Wie bei der Erläuterung der Gehäusegruppe 100 dargelegt, erstrecken sich die Federführungsstäbe 214 im schußbereiten Zustand des Granatwerfers nach vorne bis in die entsprechenden Ausbildungen einer zugehörigen Aufnahmebohrung 134 im Stahlblock 104, in der dann auch eine auf den Federführungsstab 214 aufgeschobene Pufferfeder 218 aufgenommen ist.

Diese Pufferfeder 218 kann sich entweder unmittelbar gegen den Boden der Aufnahmebohrung 134 oder gegen einen Radialvorsprung des Federführungsstabes 214 abstützen.

Beim Zurückziehen des Federführungsstabes 214 wird die Pufferfeder 218 entweder durch die vor dieser am Federführungsstab 214 ausgebildete Führungsringwulst oder durch ihre Abstützung am Federführungsstab 214 selbst mitgenommen.

Die beiden Federführungsstäbe 214 erstrecken sich durch die Federstabführungen 136 hindurch nach hinten und sind dort durch einen Spanngriff 216 fest miteinander verbunden, der sich unter den unteren Enden des rechten und linken Handgriffs 148, 146 quer bzw. horizontal erstreckt.

Um den Verschluß 202 zu spannen, wird der Spanngriff 216 hinlänglich weit horizontal nach hinten aus dem Gehäuse 102 herausgezogen und wieder bis zum Anschlag nach vorne zurückgeschoben. Dabei ergreift die eine Hand des Schützen den dieser entsprechenden Handgriff 146 oder 148 zur Abstützung, während die andere Hand den Spanngriff 216 betätigt. Somit ist ein Spannen der Waffe möglich, ohne daß sich der Schütze über die Waffe beugen muß und ohne daß auf die Waffe Kräfte aufgebracht werden, die geeignet sind, ihre gegebenenfalls vorher erfolgte Einjustierung auf ein Ziel zu beeinträchtigen.

Im Bereich des linken Endes des Spanngriffes 216 ist an diesem ein um eine vertikale Achse schwenkbarer, federnd nach außen gedrückter Auslösehebel 220 angebracht, der so angeordnet ist, daß er bei voll nach vorne geschobenem Spanngriff 216 den Rastvorsprung 150 des Gehäuses 102 sperrend hintergreift. Dabei sind die einander zugewandten Kanten von Rastvorsprung 150 und/oder Auslösehebel 220 so abgeschrägt, daß sie ineinander einrasten, wenn sie gegeneinander bewegt werden.

Der Auslösehebel ist mit einer (nicht gezeigten) Verlängerung versehen, die so am Spannhebel 216 angeordnet ist, daß sie bei dessen Ergreifen ohne weiteres mit ergriffen werden kann, so daß die vom Rastvorsprung 150 und dem Auslösehebel 220 gebildete lösbare Sperre gelöst wird und den Spannvorgang nicht behindert.

Wird der Spanngriff 216 dagegen bis ganz nach vorne geschoben und losgelassen, dann tritt diese lösbare Sperre 150, 220 in Eingriff und verhindert jegliches unerwünschte Freikommen des Spannngriffs 216.

Der Verschlußkopf 224 trägt ferner hinten an seiner Oberseite einen mittig angeordneten Kurvenhebel-Mitnehmer 222, der bevorzugt als eine um eine vertikale Achse drehbare, gehärtete Rolle ausgebildet ist.

An der Rückseite des Masseverschlusses 202 ist ferner ein Abzugsstollen 230 angeordnet, der als querverlaufende, sich nach oben erstreckende Leiste ausgebildet ist, deren Oberkante knapp unter der Längsmitte 114 liegt und deren Vorderseite eine im wesentlichen vertikal abfallende Querfläche bildet.

Der Abzugsstollen 230 ist so ausgebildet, daß er von einer Nase am vorderen Ende eines in der Abzugseinrichtung 402 um eine horizontale Achse schwenkbar gelagerten Abzugshebels 404 von oben her umgriffen wird. Wird der Abzugshebel 404 mit seiner Nase nach oben weggeschwenkt, dann gibt er den Abzugsstollen 230 und damit den Masseverschluß 202 frei, so daß dieser unter der Wirkung der Schließfedern 234 nach vorne schnellen kann.

Oberhalb des Abzugsstollens 230 ist ein hakenartiger, nach vorne offener und von oben her ergreifbarer Fangvorsprung 232 ausgebildet, der in Fig. 10 gezeigt ist und in Zusammenhang mit der Abzugseinrichtungsgruppe 400 weiter unten erläutert werden wird.

An der Unterseite des Verschlußträgers 228 ist eine Reihe in Längsrichtung hintereinanderliegender Ratschenzähne 226 angeordnet, deren vordere Zahnflanken sich vertikal erstrecken, deren Zahnspitzen horizontal abgeflacht sind und deren hintere Zahnflanken gegenüber der Horizontalen jeweils nur um einen sehr flachen Winkel, etwa 10°, geneigt sind.

Der Grund zwischen der hinteren Zahnflanke eines vorderen Ratschenzahnes 226 und der vorderen Zahnflanke eines nachfolgenden Ratschenzahnes 226 ist horizontal abgeflacht.

Der vertikale Abstand zwischen den Ratschenzähnen 226 und der am Gehäuse 102 schwenkbar angebrachten Ratschenklinke 142 ist so bemessen, daß die Ratschenklinke 142 sich unter den Ratschenzähnen 226 nur bis zu einer solchen Schräglage aufrichten kann, daß sie imstande ist, dann, wenn sie nach hinten gekippt ist, sperrend gegen eine der vorderen Zahnflanken anzuliegen, dagegen dann, wenn sie nach vorne gekippt ist, die Ratschenzähne 226 unbehindert über sich weggleiten zu lassen.

Die Länge der zahnstangenartigen Reihe von Ratschenzähnen 226 und damit des Verschlußträgers 228 ist so bemessen, daß diese Reihe voll über die Ratschenklinke 142 nach vorne oder hinten hinweggelaufen ist, wenn sich der Masseverschluß 202 in seiner vordersten oder hintersten Lage befindet.

In jeder dieser Lagen ist es der Ratschenklinke 142 somit gestattet, sich durch Federwirkung vollständig aufzurichten, so daß sie beim Rücklauf des Masseverschlusses 202 nach hinten, beim Vorlauf dagegen nach vorne gekippt wird.

In der in Fig. 1 gezeigten Stellung befindet sich der Masseverschluß 202 in seiner Auslöselage, in der er durch den Eingriff des Abzugshebels 404 in den Abzugsstollen 230 in seiner Lage festgehalten wird. Diese Auslöselage befindet sich ein wenig vor dem hintersten Ende des Rücklaufes, wo es der Ratschenklinke gestattet ist, sich ganz aufzurichten. Nun, in der Auslöselage, liegt das vordere Ende der zahnstangenartigen Reihe von Ratschenzähnen 226 von hinten her gegen die Ratschenklinke 142 an und kippt sie nach vorne.

Wird nun der Masseverschluß 202 freigegeben, dann läuft er unbehindert über die Ratschenklinke 142 hinweg, bis er in seine vorderste Lage gelangt. Hier richtet sich die Ratschenklinke 142 hinter der zahnstangenartigen Reihe wieder auf und wird beim Rücklauf nach hinten gekippt.

Wird nun aus irgendeinem Grund der Rücklauf unterbrochen, etwa weil eine Patrone mit ungenügendem Rückstoß abgefeuert wurde oder der Schütze beim Spannen des Masseverschlusses 202 behindert wurde, so daß dessen Rückwärtsbewegung schon vor der Auslöselage abgebrochen wird, dann kann der Masseverschluß 202 nicht nach vorne schnellen. Dies ist erst dann möglich, wenn die Rücklaufbewegung mittels des Spanngriffs 216 vervollständigt wurde.

Somit wird ein unerwünschtes Abfeuern verhindert, das möglicherweise, etwa beim Loslassen des Spanngriffes 216, stattfinden könnte, weil in der dann erreichten Stellung des Masseverschlusses 202 (vor der Auslöselage) der Abzugshebel 404 noch nicht in den Abzugsstollen 230 eingreifen und den Masseverschluß 202 festhalten kann.

Das Aufhalten des Masseverschlusses 202 in einer Lage vor der Auslöselage ist bei vielen Waffen, etwa den meisten Maschinenpistolen oder Maschinengewehren, zweckmäßig, ist aber bei der dargestellten Waffe darüber hinaus deshalb von grundlegender Bedeutung, weil bei dieser Waffe die Zündung der Patrone 502 nicht erst dann erfolgt, wenn sie voll in das Patronenlager 108 eingeführt wurde, sondern schon einen kurzen, genau festgelegten Zeitraum vorher, wenn sich Patrone 502 und Masseverschluß 202 in voller Bewegung befinden, wobei in an sich bekannter Weise die dann aufgebrachte kinetische Energie zum Abfangen eines Teiles des Rückstoßes dient, der durch den Abschuß der Patrone 502 entsteht.

Da aber, wie eingangs erwähnt, die Patrone 502 nicht voll ins Patronenlager 108 eingeführt werden kann, sondern um einen beträchtlichen Abstand (axiale Länge des Gurtgliedes 508) aus dem Patronenlager 108 herausragt, wenn sie gezündet wird, werden die genaue Lage des Masseverschlusses 202 und dessen eng tolerierte Geschwindigkeit jeweils zum Zündzeitpunkt zu höchst kritischen Werten. Das beschriebene Ratschengesperre 142, 226 sorgt dafür, daß die Geschwindigkeit des Masseverschlusses 202 sich beim Zünden der Patrone 502 zuverlässig innerhalb der zulässigen Toleranz befindet.

### Die Zubringergruppe 300:

Die Zubringergruppe 300 besteht aus der eigentlichen Zubringereinrichtung, ihrer Steuerung und dem Gurteinlauf; die Steuerung ihrerseits besteht aus den gehäuseseitigen Steuerelementen und den in einem Zubringerdeckel 318 angeordneten Steuerelementen.

Die gehäuseseitigen Steuerelemente bestehen aus einem Kurvenhebel 302 und einem zweiarmigen Umlenkhebel 310, die beide jeweils um eine vertikale Achse schwenkbar im Gehäuse 102 gelagert sind.

Der Kurvenhebel 302 ist aus einem nach unten offenen U-Profilstab gebildet, dessen nach oben gewandter Boden zur Gewichtserleichterung und zur Bildung von Schmu..-Aufnahmeräumen gelocht ist. Der U-Profilstab ist insgesamt, von oben betrachtet, schwach S-förmig gebogen. Seine nach unten gerichtete Höhlung bildet eine in einer horizontalen Ebene liegende, gekrümmte Steuerkurve 304, in welcher der Kurvenhebel-Mitnehmer 222, der mittig, oben und hinten auf dem Verschlußkopf 224 sitzt, nahezu spielfrei gleiten kann.

Der Kurvenhebel 302 ist an seiner Vorderseite (Oberseite seiner S-Form) an einem Lagerzapfen 306 schwenkbar gelagert, der fest, mittig und aufrecht in der Brücke 144 angebracht ist und von dieser nach oben absteht.

Bei der geradlinigen Vor- und Rückwärtsbesegung des Masseverschlusses 202 und damit des Kurvenhebel-Mitnehmers 222 läuft dieser in der Steuerkurve 304 entlang und veranlaßt somit den Kurvenhebel 302 zu einer Schwenkbewegung, deren Ablauf von der Krümmung der Steuerkurve 304 gesteuert wird.

Der Kurvenhebel 302 weist kurz hinter der Mitte seiner Längenerstreckung eine nach rechts (zum rechten Gehäusesteg 122) hin geöffnete Kurvenhebelaussparung 320 auf, die sich in den Boden und die rechte Seitenwand des Kurvenhebels erstreckt, aber die Wirkung der Steuerkurve 304 in keiner Weise beeinträchtigt.

In diese Kurvenhebelaussparung 320 greift ein Arretierhebel (nicht gezeigt) ein, der mit einem abgefederten Tastfinger (nicht gezeigt) gekoppelt ist, der vom geschlossenen Zubringerdeckel 318 niedergehalten wird.

Normalerweise befindet sich dieser Arretierhebel außer Eingriff mit der Kurvenhebelaussparung 320 und übt somit keinerlei Wirkung aus.

Wird aber der Zubringerdeckel 318 geöffnet, etwa zum Einlegen eines Patronengurtes 500 oder zum Beseitigen einer Ladehemmung, dann kann der Tastfinger federnd ausfahren und nimmt den Arretierhebel mit, der dann in die Kurvenhebelaussparung 320 eingreift und sich an deren Rand abstützt.

Läßt nun der Schütze versehentlich den Masseverschluß 202 abschlagen, dann wird dieser durch das Auflaufen des Kurvenhebel-Mitnehmers 222 gegen den Arretierhebel aufgefangen, so daß der Masseverschluß 202 nicht die Hand des Richt- oder Ladeschützen, die sich gerade im Bereich unmittelbar hinter dem Patronenlager 108 befinden kann, erreichen und verletzen kann. Die Erschütterung bei diesem. Auflaufen ist so stark, daß sie vom Schützen bemerkt wird, der dann einfach nur den Spanngriff 216 zurückzuziehen braucht. Wegen der stark abgeschrägten Hinterkanten der Ratschenzähne 226 kann der Masseverschluß 202 nach hinten bewegt werden, obwohl die Ratschenklinke 142 nach vorne gekippt ist.

Der Kurvenhebel 302 weist unmittelbar hinter der Kurvenhebelaussparung 320 einen etwa rechtwinklig nach links abstehenden Seitenschenkel 308 auf, dessen freies Ende einen abwärtsgerichteten Zapfen trägt, der passend in ein Langloch 312 im hinteren Ende des zweiarmigen Umlenkhebels 310 eingreift.

Dieser Umlenkhebel 310 ist auf der Höhe des Kurvenhebels 302 zwischen diesem und der linken Gehäusewand 126 angeordnet und erstreckt sich etwa in Längsrichtung des Gehäuses 102.

Der zweiarmige Umlenkhebel 310 ist aus zwei gleichlangen Armen gebildet, die zwischeneinander einen sehr stumpfen Winkel von etwa 165' einschließen.

In seiner Mitte ist der zweiarmige Umlenkhebel 310 schwenkbar an einem aufrechten Lagerzapfen 312 angebracht, der fest und aufrecht nach oben abstehend am linken Gehäusesteg 124 befestigt ist.

Am vorderen, freien Ende des zweiarmigen Umlenkhebels 310 ist ein Umlenkhebelzapfen 316 angebracht, der von der Oberseite des Umlenkhebels 310 nach oben absteht. Dieser Umlenkhebelzapfen 316 befindet sich ein wenig hinter der Brücke 144.

Der Gehäusedeckel 112 deckt alle gehäuseseitigen Steuerelemente (Kurvenhebel 312, Umlenkhebel 310) von oben her staubdicht ab; lediglich das vorderste Ende des Umlenkhebels 310 zusammen mit dem Umlenkhebelzapfen 316 steht nach vorne über die Vorderkante des Gehäusedeckels 112 vor.

Vor dem Gehäusedeckel 112 ist ein Zubringerdeckel 318 auf dem Gehäuse 102 angebracht und mittels einer Scharnieranordnung, die auf der Oberseite des Stahlblocks 104 ausgebildet ist, um eine horizontale Querachse schwenkbar befestigt. Der Zubringerdeckel 318 ist in Fig. 1 gezeigt, in Fig. 2 nur in seinem Umriß strichpunktiert angedeutet und in Fig. 3 schematisiert. In allen drei Figuren befindet sich der Zubringerdeckel 318 in seinem geschlossenen Zustand, in dem er durch eine lösbare Sperre gehalten wird.

Der Zubringerdeckel 318 ist um fast einen Patronendurchmesser breiter als das Gehäuse, erstreckt sich nach hinten bis über die Vorderkante des Gehäusedeckels 112 hinaus und schirmt somit wie ein Vordach die jeweilige Einführöffnung 116, 118 im Gehäuse 102 gegenüber herabfallendem Schmutz (Schlamm, Sand, Erde) ab.

Ferner verdeckt der Zubringerdeckel 318 den Spalt zwischen der Brücke 144 und der Vorderkante des Gehäusedeckels 112.

Der Zubringerdeckel 318 ist als ein nach unten offener, flacher Behälter ausgebildet. In dem Teil des Zubringerdeckels 318, der in dessen Schließstellung über der Brücke 144 liegt, sind zwei vertikale Lagerzapfen 322, 324 fest angebracht, deren Achsen mit jeweils gleichem Abstand von der Längsmitte 114 in einer gemeinsamen, zu dieser senkrechten Ebene liegen.

Auf dem linken Lagerzapfen 324 ist ein im wesentlichen gerader, erster Steuerhebel 326 schwenkbar gelagert, der in sich der in Fig. 2 gezeigten Lage des Masseverschlusses 202 (Auslöselage) um einen Winkel von etwa 15° vom Lagerzapfen 324 aus nach vorne und auswärts erstreckt.

Der erste Steuerhebel 326 ist nach hinten verlängert und endet in einem hinteren Aufnahmemaul 336, das in lösbarem und kraftübertragendem Eingriff mit dem Umlenkhebelzapfen 316 steht.

Auch am vorderen Ende weist dieser erste Steuerhebel 326 ein Aufnahmemaul 338 auf, das in lösbarem kraftübertragendem Eingriff mit einem ersten Schieberzapfen 346 steht.

Der erste Steuerhebel 326 weist auch noch einen im wesentlichen horizontal und rechtwinklig vom Bereich des linken Lagerzapfens 324 abstehenden Steuerhebelschenkel 330 auf, der bis etwa über die Längsmitte 114 reicht und an seinem freien Ende einen sich vertikal nach unten erstreckenden Eingriffszapfen 334 trägt.

Auf dem rechten Lagerzapfen 322 ist ein im wesentlichen gerader, zweiter Steuerhebel 328 schwenkbar gelagert, der in sich der in Fig. 2 gezeigten Lage des Masseverschlusses 202 (Auslöselage) um einen Winkel von etwa 15° vom Lagerzapfen 324 aus nach vorne und auswärts erstreckt, und zwar symmetrisch zum ersten Steuerhebel 326.

Am vorderen Ende weist dieser zweite Steuerhebel 328 ein Aufnahmemaul 358 auf, das in lösbarem kraftübertragendem Eingriff mit einem zweiten Schieberzapfen 348 steht.

Der zweite Steuerhebel 328 ist etwa rechtwinklig abgewinkelt, wobei der Scheitel des Winkels im Bereich des rechten Lagerzapfens 322 liegt.

Der abgewinkelte Teil des zweiten Steuerhebels 328 bildet einen Steuerhebelschenkel 330, der bis etwa über die Längsmitte 114 reicht und an seinem freien Ende ein Langloch 332 aufweist, das den Eingriffszapfen 334 mit Gleitpassung aufnimmt und sich im wesentlichen quer zu dessen Bewegungsbahn beim Verschwenken des ersten Steuerhebels 326 erstreckt.

Der Eingriffszapfen 334 und das Langloch 332 bilden somit eine im wesentlichen spielfreie Zwangskoppelung, die dafür sorgt, daß der zweite Steuerhebel 328 genau gegenläufig der Bewegung des ersten Steuerhebels 326 bei dessen Schwenkbewegung folgt: schwenkt beispielsweise der hintere Teil des Kurvenhebels 302 in der Draufsicht der Fig. 2, in Schußrichtung gesehen, nach rechts, dann bewegen sich die beiden vorderen Aufnahmemäuler 338 der beiden Steuerhebel 326, 328 aufeinander mit gleicher Geschwindigkeit zu.

Die beiden Schieberzapfen 346, 348 (Fig. 3) sind bevorzugt als drehbar gelagerte Rollen ausgebildet, um die Reibung beim Eingriff in die Aufnahmemäuler 338 zu mindern.

Der Zubringerdeckel 318 nimmt aber nicht nur einen Teil der Steuerung, wie beschrieben, sondern auch den wesentlichen Teil der eigentlichen Zubringereinrichtung auf.

Diese weist einen ersten Schieber 342 und einen zweiten Schieber 344 (Fig. 4) auf, die beide horizontal und quer zur Längsmitte 114 verschieblich in einer Schieberführung 340 aufgenommen sind, die im Zubringerdeckel 318 enthalten ist.

Der erste Schieber 342 trägt nach oben abstehend den ersten Schieberzapfen 346, der zweite Schieber 344 (Fig. 4) ebenso den zweiten Schieberzapfen 348. Die beiden Schieberzapfen und deren Bewegungsbahnen liegen beide auf einer gemeinsamen, zur Längsmitte 114 senkrechten Ebene.

Diese Schieberführung 340 ist in ihrem Querschnitt quer zur Richtung der Schieberbewegungen so ausgebildet und ist soweit zerlegbar, daß die beiden Schieber 342, 344 herausgenommen und entgegen ihrer ursprünglichen Bewegungsrichtung wieder eingesetzt werden können.

Infolge des symmetrisch erfolgenden Antriebs durch die beiden Steuerhebel 326, 328 funktionieren die beiden Schieber 342, 344 auch in umgekehrter Ausrichtung, fördern dann aber den Patronengurt 500 in Gegenrichtung in die Waffe, also nicht durch die linke Einführöffnung 118, wie in Fig. 2 gezeigt, sondern durch die rechte Einführöffnung 116.

Die jeweils nicht benutzte Einführöffnung 116, 118 wird, wie aus Fig. 3 ersichtlich, von einer Blechplatte 350 oder sonstigen Abdeckung verschlossen, um das Eindringen von Schmutz in die Waffe zu verhindern.

Die weiteren Elemente der Zubringereinrichtung werden unter Bezugnahme auf die Fig. 4 bis 8 beschrieben und sind der Deutlichkeit halber nur in diesen Figuren mit Bezugszeichen versehen.

Der erste Schieber 342 weist, nach unten abstehend, am rechten Ende einen festen Anschlag 356 auf, am linken Ende eine Außenklinke 352 und etwa in der Mitte eine Innenklinke 354.

Der zweite Schieber 344 weist, nach unten abstehend, am rechten Ende eine feste Stütze 360 und am linken Ende eine Schwenkklinke 358 auf.

Die Klinken 352, 354 und 358 sind jeweils als nach unten vorstehende Finger ausgebildet, die an ihrem oberen Ende jeweils gegen die Kraft einer Federung nach oben und zu der durch die Blechplatte 350 verschlossenen Einführöffnung 116 hin jeweils um eine Achse schwenkbar sind, die parallel ist zur Längsmitte 114.

Durch die andere Einführöffnung 118 läuft der Patronengurt 500 in die Waffe hinein.

Die Unterkanten der Klinken 352, 354 und 358 sind so ausgebildet, daß sie, wenn sie nach unten vorstehen und in Einlaufrichtung des Patronengurtes 500 bewegt werden, dessen jeweils vorderste und gegebenenfalls zweite Patrone 502 und 504 hintergreifen und fördern.

Werden die Klinken 352, 354 und 358 jedoch entgegen der Einlaufrichtung bis an eine Patrone 504 heranbewegt, dann werden sie durch die jeweils auflaufende Patrone weggeschwenkt, so daß diese unter ihnen passieren kann.

Lediglich die Außenklinke 352 gelangt dann, wenn sich die beiden Schieber 342, 344 zwischen den beiden Relativlagen der Figuren 3 und 4 befinden, in Sperreingriff mit dem zweiten Schieber 344, so daß sie dann nicht wegschwenken kann, sondern entgegen der Einlaufrichtung des Patronengurtes 500 gegen die zweite Patrone 504 anläuft und sie (und damit den ganzen Patronengurt 500) ein wenig zurückschiebt, ohne wegzuschwenken.

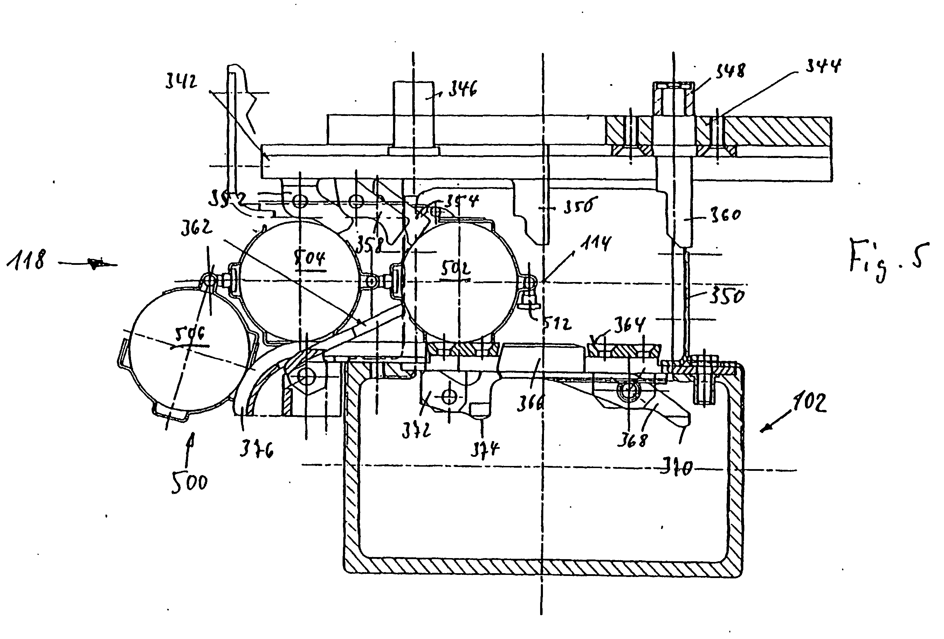

Am Gehäuse 102 ist unter der benutzten Einführöffnung 118 ein in diese hinein weisender Sperrhebel 362 mit seinem außenliegenden Ende um eine zur Längsmitte 114 parallele Achse schwenkbar gelagert, wird durch eine Federung bis in die in Fig. 4 und 5 gezeigte Lage angehoben und kann durch die über ihm weglaufenden Patrone 504 in die in Fig. 6 bis 8 gezeigte Lage niedergedrückt werden.

Die Wirkungsweise der von den beiden Schiebern 344, 346 getragenen Elemente und des Sperrhebels 362 wird nachfolgend kurz anhand der in den Figuren 4 bis 8 gezeigten Bewegungsfolge erläutert:

Fig. 4 zeigt die Lage des Patronengurtes 500 und der beiden Schieber 344, 346, wenn die Waffe nach Abgabe eines Schusses gespannt und schußbereit ist, sich der Masseverschluß 202 demnach in seiner Auslöselage befindet.

Der Sperrhebel 362 ist aufgestellt und stützt die erste Patrone 502 von außen her ab, die Schwenkklinke 358 ist gerade dabei, über diese Patrone 502 hinwegzulaufen und hintergreift sie bereits, hat aber noch nicht ihre voll aufrechte Lage erreicht. Die Außenklinke 352 wird gerade von der zweiten Patrone 504 nach oben weggeschwenkt, und die Innenklinke 354 befindet sich in voll aufrechter Lage.

Soll der Patronengurt 500 dagegen erst eingelegt werden, dann wird der Zubringerdeckel 318 aufgeklappt, wobei sich dann alle Klinken 352, 354 und 358 in voll aufrechter lage befinden, der Patronengurt 500 wird mit seiner vordersten Patrone 502 hinter das aufstehende, freie, zur Längsmitte 114 hin weisende Ende des Sperrhebels 362 gelegt und gegen dieses durch leichtes Anziehen am Patronengurt 500 angehalten und der Zubringerdeckel 318 wird wieder geschlossen.

Dann ist die Lage aller Elemente dieselbe wie in Fig. 4, mit der Ausnahme, daß sich die Schwenkklinke 358 in voll aufrechter Lage befindet und die erste Patrone 502 hintergreift.

Beginnt nun der Masseverschluß 202 mit seiner Vorwärtsbewegung, dann begingen die beiden Schieber 342, 344 eine solche Bewegung, daß sich die beiden Schieberzapfen 346, 348 aufeinander zu bewegen, bis sie die in Fig. 5 gezeigte Lage erreichen.

Die Innenklinke 354 hat sich mittlerweile in Einlaufrichtung des Patronengurtes 500 gegen die erste Patrone 502 heranbewegt, die Schwenkklinke 358 entgegen der Einlaufrichtung wegbewegt.

Bei der weiteren Bewegung schiebt die Innenklinke 354 die erste Patrone 502 bis vor das Patronenlager 108 (siehe Fig. 3), während die Schwenkklinke 358 nach außen über die zweite Patrone 504 hinwegläuft. Die feste Stütze 360 ist bis an die erste Patrone herangelaufen, die zwischen dieser festen Stütze 360, der Innenklinke 354 und Patronenauflagefingern 366, die weiter unten erläutert werden, in einer genau definierten Lage festgehalten wird. Die voll aufgerichtete Aussenklinke 352 liegt gegen die Seite der zweiten Patrone 504 an, die der ersten Patrone 502 zugewandt ist.

Der Abstand der beiden Schieberzapfen 346, 348 hat sein Minimum erreicht.

Nun erreicht der Verschlußkopf 224 mit dem Stoßboden 208 den Patronenboden und schiebt die erste Patrone 502 nach vorne.

Dabei verschiebt sich der Anlenkzapfen 512 am Gurtglied 508 der zweiten Patrone 504 im Langloch des Vorsprungs 510 am Gurtglied 508 der ersten Patrone. Die beiden Schieber 342, 344 kehren ihre Bewegungsrichtung um und beginnen, sich mit ihren Schieberzapfen 346, 348 auseinanderzubewegen.

In dieser Relativlage der beiden Schieber 342, 344 übergreift, wie bereits oben erörtert, der zweite Schieber 344 die Außenklinke 352 und hindert sie daran, zu schwenken.

Die Außenklinke 352 schiebt somit die zweite Patrone 504 entgegen der Einlaufrichtung von der ersten Patrone 502 weg, wobei der Anlenkzapfen 512 des Gurtglieds 508 der zweiten Patrone 504 aus der Erweiterung im Langloch des zugewandten Vorsprungs 510 herausgezogen wird.

Die zweite Patrone 504 bewegt sich weiter nach außen, bis sie an der Schwenkklinke 358 zum Stillstand gelangt (Lage der Fig. 7). Hierbei hat die zweite Patrone dem vorbeilaufenden Verschlußkopf 224 Platz gemacht, ebenso wie die Innenklinke 354 und die feste Stütze 360, die beide von der ersten Patrone 502 zurückgefahren sind, um den Verschlußkopf 224 vorbeizulassen. Die seitliche Abstützung der Patrone 502 ist nun nicht mehr nötig, da sie sich mit dem vorderen Teil bereits im Patronenlager 108 befindet und mit dem Patronenboden am Stoßboden 208 gehalten ist.

Wenn die Patrone 502 abgefeuert wird, dann befinden sich alle Elemente der Zubringereinrichtung in der in Fig. 7 gezeigten Lage.

Nun beginnt der Verschlußrücklauf, und die bisher beschriebene Bewegungsfolge läuft in umgekehrter Reihenfolge wieder ab.

Beim Ausziehen der abgeschossenen Patronenhülse nähern sich die Innenklinke 354 und feste Stütze 360 an diese an und führen sie,

Dann bewegen sich die beiden Schieber 342, 344 mit ihren Schieberzapfen 346, 348 rasch auseinander, wobei die Schwenkklinke 358 die bisher zweite Patrone 504 und jetzt erste Patrone 502 bis in die Lage der Fig. 4 nachführt, wo sie vom Sperrhebel 362 hintergriffen wird.

Dabei verhindert der feste Anschlag 356 oder 360, daß die Patrone 502 zu weit gefördert wird.

An der Außenseite des Gehäuses 102 ist unter der linken Einlauföffnung 118 eine nach außen und unten gekrümmte Gurtführungsbühne 376 angebracht. Wird die rechte Einlauföffnung 116 für den Gurteinlauf benutzt, dann kann sie aufgrund ihres symmetrischen Aufbaus auch abgenommen, umgedreht und unter der rechten Einlauföffnung 116 angebracht werden.

Auch der Sperrhebel 362 kann vor der rechten Einlauföffnung 116 angebracht werden.

An die Gurtführungsbühne 376 schließt im Gehäuse 102 höhengleich ein horizontaler Führungstisch 364 an, der hinter dem Patronenlager 108 einen Durchbruch aufweist, der von einem höhengleichen Patronenauflagefinger 366 überbrückt ist.

Dieser ist rechts unter der angrenzenden Kante des Führungstisches 364 an einer zur Längsmitte 114 parallelen Achse schwenkbar gelagert und wird durch eine Feder nach oben gedrückt, wobei der genannte Durchbruch im Führungstisch 364 seine obere Endlage festlegt.

Der Patronenauflagefinger 366 ist nach rechts unten über die Lagerung hinaus durch einen Führungshebel 368 verlängert, dessen Ende einen Führungshebel-Mitnehmer 370 bildet.

Dieser Führungshebel-Mitnehmer 370 ist in Zuordnung zur Bewegung des Masseverschlusses 202 so angeordnet, daß dann, wenn die vorderste Patrone 502 in das Patronenlager 108 eingeführt werden soll, der Verschlußträger 228 (in Fig. 7 gestrichelt gezeigt) gegen den Führungshebel-Mitnehmer 370 anläuft und dabei den Patronenauflagefinger 366 soweit nach unten wegschwenkt (Fig. 7), daß sich die Patrone 502 trotz ihres überstehenden Randes und trotz des unteren, abgeflachten Vorsprungs 514 des Gurtglieds 508 genau koaxial zum Patronenlager 108 und damit zur Längsmitte ausrichten kann.

Um zu verhindern, daß der Patronenauflagefinger 366 unter der Einwirkung von Stößen unkontrolliert nach unten schwingt, ist unter dem linken Rand des genannten Durchbruchs im Führungstisch 364 ein Klemmhebel 372 um eine zur Längsmitte 114 parallele Achse schwenkbar angebracht, der das freie Ende des Patronenauflagefingers 366 untergreift und damit festlegt.

Der Klemmhebel 372 ist an seiner Unterseite mit einem Klemmhebel-Mitnehmer 374 versehen, der vom Verschlußträger 228 ebenso wie der Führungshebel-Mitnehmer 370 hochgedrückt werden kann, um den Führungshebel 368 freizugeben (Fig. 7).

Wie ersichtlich, ist der Patronenauflagefinger 366 nur dann weggeschwenkt, wenn die Patrone 502 gerade in das Patronenlager 108 eingeführt oder deren Patronenhülse aus diesem ausgezogen wird.

Alle oben beschriebenen Elemente, die in unmittelbare Berührung mit dem Patronengurt 500 gelangen, sind vorzugsweise mindestens zweimal in Längsrichtung der Waffe nebeneinanderliegend angeordnet, um sicherzustellen, daß die Patronen 502, 504, 506 während des gesamten Zubringebetriebes stets parallel zur Längsmitte 114 ausgerichtet sind und bleiben.

Beiderseits der für den Gurteinlauf benutzten Einführöffnung 118 befindet sich, wie in Fig. 2 gezeigt, eine um eine aufrechte Achse drehbar gelagerte Patronengurt-Führungsrolle 378, deren Durchmesser etwa dem einer Patrone 502, 504, 506 entspricht. Hierdurch wird ein sauberer Gurteinlauf gewährleistet.

Diese Patronengurt-Führungsrollen 378 können auch an der anderen Einführöffnung 116 angebracht werden.

### Die Abzugseinrichtungsgruppe 400:

Die Abzugseinrichtungsgruppe weist die eigentliche Abzugseinrichtung 402 auf, die in einem gehäuseartigen Kasten untergebracht ist, der an der Rückseite der Endabdeckung 110 des Gehäuses 102 angebracht ist und zwischen den beiden Handgriffen 146, 148 sitzt.

Beiderseits des Kastens ist in ergonomischer Zuordnung zu den Handgriffen 146, 148 jeweils eine Daumenplatte 406 angebracht, die als Abzug dient und mit dem Abzugshebel 404 so verbunden ist, daß sich beim Niederdrücken einer oder beider der Daumenplatten 406 der Abzugshebel 404 mit seinem freien Ende hebt, dabei den Abzugsstollen 230 freigibt und somit den Masseverschluß 202 nach vorne schnellen läßt.

Unterhalb der Daumenplatte sitzt an einer oder jeder Seitenwand des Kastens ein Sicherungs- und Feuerwahlhebel, der drehfest auf einer Welle 408 (Fig. 10) angebracht ist.

Der Sicherungs- und Feuerwahlhebel hat ebenso wie die Welle 408 drei Drehlagen: S (Sicher), E (Einzelfeuer) und D (Dauerfeuer). Die in Fig. 10 gezeigte Stellung ist die Drehlage S (Sicher).

Der Aufbau der zugeordneten Sicherungs- und Feuerwahleinrichtung ist herkömmlich und hier nicht gezeigt; in der Drehlage S sind die Daumenplatten 406 und der Abzugshebel 404 arretiert, in den anderen Drehlagen freigegeben; zusätzlich wird in der Drehlage E nach einmaligem Schwenken des Abzugshebels 404 die Verbindung zwischen diesem und den Daumenplatten 406 unterbrochen, so daß der Abzugshebel 404 nach Auslösung eines Schusses seine Abzugsstollen-Haltelage wieder einnehmen kann, auch wenn die Daumenplatten 406 niedergedrückt bleiben; in der Drehlage D sind Daumenplatten 406 und Abzugshebel 404 ständig fest bewegungsverbunden.

Zusätzlich zur beschriebenen, bekannten Sicherungseinrichtung hat aber die Welle 408 zusätzlich einen unrunden, in Fig. 10 gezeigten Steuerabschnitt, der vom gegabelten Ende des einen Schenkels (Abstützschenkels) 418 eines Sicherungs-Winkelhebels 424 umgriffen ist.

In der gezeigten Sicherungslage S wird der gegabelte Abstützschenkel 418 mit seiner hinteren Endkante gegen einen Anschlag 420 angedrückt. In der Einzelfeuer- und Dauerfeuerlage E und D wird dagegen der Abstützschenkel 418 durch den unrunden Steuerabschnitt der Welle 408 vom Anschlag 420 wegbewegt.

Der Sicherungs-Winkelhebel 424 ist im Bereich seines Scheitels schwenkgelagert und weist als zweiten Schenkel einen Fanghaken 412 auf, der sich in der Sicherungslage S nach vorne bis über den Fangvorsprung 232 des Masseverschlusses 202 hinaus erstreckt und diesen umgreift.

Auf dem Sicherungs-Winkelhebel sitzt ferner ein Nocken 410, der eine Abflachung aufweist, die in der gezeigten Sicherungslage S flächig gegen eine Druckplatte 422 anliegt, die ihrerseits schwenkbar gelagert ist und von einer Feder gegen den Nocken 410 hin belastet ist.

Wie in Fig. 10 gezeigt, haben der Fangvorsprung 232 und das freie Ende des Fanghakens 412 eine komplementäre Ausbildung, so daß sie sich fest gegenseitig hintergreifen und miteinander verhaken können, wenn der Masseverschluß 202 etwa infolge eines Bruchs des Abzugshebels 404 trotz der Wahl der Sicherungslage S beginnt, sich nach vorne zu bewegen.

Im Gegensatz zur Drehlage S ist in den Drehlagen E und D der Welle 408 der Sicherungs-Winkelhebel 424 so verschwenkt, daß das hakenartig gekrümmte freie Ende des Fanghakens 412 aus der Bewegungsbahn des Fangvorsprungs 232 herausgehoben ist und die freie Bewegung des Masseverschlusses 202 nicht behindert.

Bricht allerdings der Abstützschenkel 418, so daß der Sicherungs-Winkelhebel nicht mehr auf die Drehlage der Welle 408 anspricht, dann bringt die Druckplatte 422 den Nocken 410 und damit den Fanghaken 412 in die gezeigte Sicherungslage S.

Infolge der Formgebung der Hakenanordnung ist bei deren Eingriff der Fanghaken 412 festgelegt und damit die Welle 408 blockiert, so daß es nicht möglich ist, die Waffe zu entsichern und somit ungewollt gleichzeitig abzufeuern.

Die voranstehend beschriebene, eigentliche Abzugseinrichtung löst den Masseverschluß 202 aus, nicht aber den eigentlichen Zündvorgang. Dieser wird von der in Fig. 9 schematisch dargestellten Zündeinrichtung ausgelöst, und zwar in Zuordnung zur präzisen Lage des Masseverschlusses 202; es wurde weiter oben bereits darauf hingewiesen, daß bei der erfindungsgemäßen Waffe das Einhalten eines genau definierten Zündzeitpunktes innerhalb engster Toleranzen besonders wichtig ist.

Wie bereits bei der Beschreibung der Gehäusegruppe 100 erläutert, erstreckt sich längs der Bewegungsbahn des Verschlußkopfes 224 am rechten Gehäusesteg 122 eine Steuerkurve 138 für die Schlagbolzenhülse 416 und am linken Gehäusesteg 124 eine Steuerkurve 140 für den Schlagbolzen 414.

Die Schlagbolzenhülse 416 weist einen stabförmigen, vorderen Teil und einen kolbenartigen, hinteren Teil auf, die in der mit einem entsprechenden Durchmesser bemessenen Axialbohrung 212 des Verschlußkopfes 224 hin- und herbeweglich aufgenommen ist.

Beide Teile sind von einer Hülsen-Längsbohrung 426 durchsetzt, mit einem vorderen, engen Durchlaß für die Schlagbolzenspitze, einem Hauptabschnitt für den Schlagbolzenschaft und einem erweiterten Endabschnitt zur Aufnahme des verdickten Schlagbolzenendes.

Am Außenumfang des erweiterten Endabschnitts befindet sich eine als Führungshebel-Aufnahme 428 ausgebildete Vertiefung.

Der Schlagbolzen 414 weist, wie bereits angedeutet, eine Schlagbolzenspitze, einen schlanken, mit Führungs-Ringvorsprüngen versehenen Schlagbolzenschaft und ein verdicktes Schlagbolzenende mit einer nach hinten offenen Sackbohrung auf, die zur Aufnahme einer Schlagfeder (nicht gezeigt) ausgebildet ist.

An der Außenseite des verdickten Schlagbolzenendes ist an diesem ein durchbohrter Quervorsprung ausgebildet; die zu ihrem Ende hin konisch erweiterte Bohrung des Quervorsprungs bildet eine Spannhebel-Aufnahme 434.

Das hintere Ende der Axialbohrung 212 im Verschlußkopf 224 ist von einer Federabstützbüchse 444 verschlossen, auf deren Boden sich die in der Sackbohrung im verdickten Schlagbolzenende aufgenommene Schlagfeder abstützt.

Der Verschlußkopf 224 ist von oben her bis zu seiner Axialbohrung 212 an den Stellen, an denen die Bewegungsbereiche der Führungshebel-Aufnahme 428 und der Spannhebel-Aufnahme 434 liegen, geschlitzt; innerhalb der so gebildeten Schlitzanordnung liegen hintereinander drei an einer jeweils zugehörigen, horizontalen Querachse im Verschlußkopf 224 gelagerte Steuerelemente.

Das vorderste dieser Steuerelemente ist ein Führungshebel 430, der wie eine Wiege geformt ist und mit seinen beiden vorspringenden Enden an der Steuerkurve 138 für die Schlagbolzenhülse 416 entlangläuft.

Wie ersichtlich, ist die Kipplage des Führungshebels 430 abhängig von der Formgebung der Steuerkurve 138.

Der Führungshebel 430 weist einen rechtwinklig abstehenden, fest angebrachten Mitnahmefinger 432 auf, dessen kugelig verdicktes freies Ende in der Führungshebel-Aufnahme 428 sitzt.

Die Kipplage des Führungshebels 430 bestimmt somit zwangsweise die axiale Lage der Schlagbolzenhülse 416.

Die Steuerkurve 138 ist so ausgebildet, daß der Führungshebel 430 nur in jenem Bereich der Verschlußbewegung seine vordere Lage einnehmen kann, in dem auch die Zündung erfolgen soll. Da aber der von Teilen der Axialbohrung 212 und der Hülsen-Längsbohrung 426 gebildete Durchlaß für die Spitze des Schlagbolzens 414 nur dann kurz genug ist, um die Schlagbolzenspitze in einer für die Zündung ausreichenden Länge hindurchtreten zu lassen, wenn sich die Schlagbolzenhülse 416 in ihrer vorderen Lage befindet, ist nur in dem vorgeschriebenen engen Bereich der Verschlußbewegung, in dem die Zündung erfolgen muß, eine solche Zündung überhaupt möglich.

Das mittlere Steuerelement ist ein Spannhebel 436, der wie der Führungshebel 430 wiegenartig ausgebildet ist und an der Steuerkurve 140 entlangläuft, die seine Kipplage um seine Lagerung erzwingt.

Im Gegensatz zum Führungshebel 430 ist am vorderen, an der Steuerkurve 140 ablaufenden Ende des Spannhebels 436 eine Rolle angebracht, die die beim Spannen der Schlagfeder aufzubringenden Kräfte überträgt.

Das hintere Ende des Spannhebels 436 ist durch eine Rastvertiefung ausgespart, die der Drehachse des dritten Steuerelements (das weiter unten beschrieben wird) zugewandt ist.

Der Spannhebel weist einen etwa rechtwinklig abstehenden, fest angebrachten Spannfinger 440 auf, mit einem kugeligen freien Ende, das in der Spannfinger-Aufnahme 434 sitzt.

Angesichts der hohen, beim Spannen der Schlagfeder zu übertragenden Kräfte sind der Spannfinger 440 und die Spannfinger-Aufnahme 434 größer bemessen als der Mitnahmefinger 432 und die Mitnahmefinger-Aufnahme 428.

Das dritte, hinterste Steuerelement ist ein Auslöser 442, der als zweiarmiger Winkelhebel ausgebildet ist, dessen einer (hinterer) Arm gegen die Steuerkurve 140 oder eine eigene Steuerkurve angedrückt wird und auf dieser abläuft; der andere (vordere) Arm weist an seinem freien Ende eine Rastnase 438 auf, die bei gespannter Schlagfeder in die Rastvertiefung an der Rückseite des Spannhebels 436 einfällt.

Wie ersichtlich, kann die Steuerkurve 140 eine Kippbewegung des Auslösers 442 veranlassen, dessen Rastnase dann aus der Rastvertiefung herausgeschwenkt wird, woraufhin der Spannhebel freigegeben wird und die Schlagfeder abschlagen kann, vorausgesetzt, die örtliche Ausbildung der Steuerkurve 140 läßt dies zu.

In Fig. 9 ist die Position der Zündeinrichtung gezeigt, die sie ganz kurz vor der Zündung einnimmt, also am vorderen Ende der Steuerkurven 138, 140.

Der Führungshebel 430 hat bereits die Kipplage eingenommen, in welcher er die Schlagbolzenhülse 416 in ihre vorderste Lage versetzt hat. Der Spannhebel ist bereits über die vorderste Abschrägung der Steuerkurve 140, die sein Kippen zum Spannen der Schlagfeder veranlaßt, nach vorne hinweggelaufen, liegt aber nicht gegen diese Steuerkurve 140 an, da er vom Auslöser 442 über den Eingriff Rastvertiefung/Rastnase in seiner Lage gehalten wird. Wenn dieser Auslöser, was unmittelbar bevorsteht, von der Steuerkurve 140 nach hinten gekippt wird, dann kann der Spannhebel kippen, die Schlagfeder kann sich entspannen und der Schlagbolzen kann nach vorne schnellen und die Patrone zünden.

## Patentansprüche

1. Selbstlade-Granatwerfer mit
- einer Patronengurt-Zuführeinrichtung (300), die mittels ind en Patronengurt (500) eingreifender Klinken eine Patrone bevorzugt in horizontaler Richtung bis vor ein Patronenlager fördert, wobei die Klinken (352, 354, 358) von zwei in einem aufklappbaren Zubringerdeckel (218) angeordneten, gegenläufigen Schiebern (342, 344) getragen sind und nach unten abstehen,
- einem Masseverschluß (202), der aus einer Auslöselage durch die Federkraft einer, vorzugsweise zweier Schließfedern (234) nach vorne längs eines Bewegungsweges gegen das Patronenlager (108) läuft und dazu eingerichtet ist, bei diesem Vorlauf die von den Klinken (352, 354, 358) geförderte Patrone (502, 504, 506) aus dem Patronengurt (500) in das Patronenlager (108) zu schieben sowie nach deren Zündung durch den entstehenden Rückstoß den Bewegungsweg in einem Rücklauf zurückzulegen und dabei die Schließfeder(n) (234) zu spannen,
- einer mit dem Masseverschluß (202) und den Schiebern (342, 344) verbundenen Steuerung (302, 390), die den Vor- und Rücklauf des Masseverschlusses (202) in die hierzu quergerichtete Wechselbewegung der Schieber (342, 344) umsetzt,
- einer Zündeinrichtung mit einem Schlagbolzen (414) der
- vorzugsweise durch eine Schlagfeder - gespannt und in gespanntem Zustand durch eine Arretierung gehalten ist, wobei die Arretierung (442) gelöst und die Patronen (502, 504, 506) gezündet wird,
- einer Pufferfedereinrichtung (218) zum Verzögern der letzten Phase des Vorlaufes, um beim Leerabschlagen des Granatwerfers dessen Beschädigung zu vermeiden,
- zwei im Gehäuse (102) angeordneten Federführungsstäben (214), die jeweils eine Längsbohrung (206) im Masseverschluß (202) durchsetzen und, auf denen jeweils eine der Schließfedern (234) sitzt,
wobei zur Bildung der Pufferfedereinrichtung am vorderen Ende eines jeden Federführungsstabes (214) jeweils eine gegen das Gehäuse (102) abgestützte Pufferfeder (218) angeordnet ist, die bevorzugt über den größten Teil ihrer Länge in einer vom zugehörigen Federführungsstab (214) durchsetzten Aufnahmebohrung (134) sitzt.

## Claims

1. A self-loading grenade launcher having
- a cartridge belt feed device (300), which by means of pawls engaging in the cartridge belt (500) conveys a cartridge preferably in the horizontal direction until it is in front of a cartridge chamber, wherein the pawls (352, 354, 358) are borne by two counteracting slides (342, 344) disposed in a hinged carrier cover (218) and project downwards,
- an inertia bolt (202), which from a release position runs forward along a path of motion under the spring force of one, preferably two return springs (234) towards the cartridge chamber (108) and is set up to slide the cartridge (502, 504, 506) conveyed by the pawls (352, 354, 358), during this forward movement, out of the cartridge belt (500) into the cartridge chamber (108) and also after its firing by the resultant blowback to run back along the path of motion in a recoil movement and in so doing to tension the return spring(s) (234),
- a control system (302, 390) connected to the inertia bolt (202) and the slides (342, 344), which converts the forward and recoil movement of the inertia bolt (202) into the alternating movement of the slides (342, 344) directed transversely thereto,
- a firing device with a firing pin (414) which is tensioned - preferably by a main spring - and is held by a detent in the tensioned state, whereby the detent (442) is released and the cartridges (502, 504, 506) are fired,
- a buffer spring mechanism (218) to delay the last phase of the forward movement so as to prevent the grenade launcher being damaged if it is tripped while empty,
- two spring guide rods (214) disposed in the housing (102), which each pass through a longitudinal bore (206) in the inertia bolt (202) and on each of which one of the return springs (234) is seated,
wherein to form the buffer spring mechanism a buffer spring (218), which is supported against the housing (102) and which over the greatest part of its length is preferably seated in a housing bore (134) through which the associated spring guide rod (214) passes, is disposed on the front end of each spring guide rod (214).

## Revendications

1. Lance-grenades à chargement automatique comprenant :
- un dispositif d'introduction (300) de la bande de cartouches qui, au moyen de cliquets venant en prise dans la bande de cartouches (500), transporte une cartouche, de préférence dans le sens horizontal, jusque devant un magasin de cartouches, où les cliquets (352, 354, 358) sont supportés par deux coulisseaux (342, 344) mobiles en sens contraire et disposés dans un couvercle d'amenée relevable (218), et sont en saillie vers le bas,
- une culasse (202) qui, depuis une position de déclenchement, sous l'effet de la force d'un ressort de fermeture, de préférence de deux ressorts de fermeture (234), se déplace vers l'avant, le long d'une trajectoire en direction du magasin de cartouches (108), et est agencée pour, lors de ce mouvement vers l'avant, pousser la cartouche (502, 504, 506) amenée par les cliquets (352, 354, 358), pour qu'elle sorte de la bande de cartouches (500) et entre dans le magasin de cartouches (108), ainsi que, après la mise à feu de cette cartouche, sous l'effet de recul se produisant, revenir en arrière en suivant la trajectoire et, ce faisant, bander le ou les ressort(s) de fermeture (234),
- un organe de commande (302, 390) relié à la culasse (202) et aux coulisseaux (342, 344), lequel organe de commande convertit le mouvement de la culasse (202) se produisant vers l'avant et vers l'arrière, en mouvement alterné des coulisseaux (342, 344), effectué de façon transversale par rapport au mouvement vers l'avant et vers l'arrière,
- un dispositif de mise à feu comprenant un percuteur (414) qui est armé, de préférence, par un ressort percuteur et maintenu à l'état armé par une détente,
où la détente (442) est débloquée et la cartouche (502, 504, 506) mise à feu,
- un dispositif à ressort amortisseur (218) servant à retarder la dernière phase du mouvement vers l'avant, pour, lors de la percussion à vide du lance-grenades, éviter son endommagement,
- deux tiges de guidage de ressort (214) disposées dans le carter (102), lesquelles tiges de guidage de ressort traversent à chaque fois un perçage longitudinal (206) pratiqué dans la culasse (202) et sur lesquelles est placé, à chaque fois, l'un des ressorts de fermeture (234),
où, pour la formation du dispositif à ressort amortisseur, au niveau de l'extrémité avant de chacune des tiges de guidage de ressort (214), un ressort amortisseur (218) est disposé à chaque fois en venant en appui contre le carter (102), lequel ressort amortisseur se positionne, de préférence sur la majeure partie de sa longueur, dans un perçage de réception (134) traversé par la tige de guidage de ressort respective (214).
